# EUROPÄISCHE PATENTANMELDUNG

(11) **EP 3 616 593 A1**
(43) Veröffentlichungstag der Anmeldung: **04.03.2020**
(21) Anmeldenummer: 19190241.0
(22) Anmeldetag: 06.08.2019
(51) Int. Cl.: A61B 1/00, A61B 1/005, A61M 25/00, G02B 23/24

(54) **ENDOSKOPSCHAFT MIT EINEM SCHICHTFÖRMIGEN AUFBAU UND VERFAHREN ZUR HERSTELLUNG EINES SOLCHEN**

(30) Priorität: 30.08.2018 DE 102018121206
(71) Anmelder: Karl Storz SE & Co. KG, 78532 Tuttlingen (DE)
(72) Erfinder: Attinger, Juerg, CH-8260 Stein am Rhein (CH)

(57) **Zusammenfassung**

Die Erfindung betrifft einen Endoskopschaft 1 mit schichtförmigem Aufbau. Bei diesem als gekrümmter oder nicht gekrümmter Hohlzylinders gebildeten Schaft 1 folgen verschiedene Schichten, sich umschließend, radial über die gesamte oder einen wesentlichen Teil der Länge des Schaftes aufeinander. Der Schaft zeigt eine innere Schicht 2 aus Kunststoff und eine die innere Schicht 2 umschließende äußere Schicht 3 aus Kunststoff sowie eine strukturgebende Schicht 4 im Übergangsbereich 5 der inneren und der äußeren Schicht 3. Dabei sind die Kunststoffe der inneren und äußeren Schicht 3 unterschiedlich und die innere und die äußere Schicht 3 im Übergangsbereich 5 ineinander verzahnt. So können sich die innere und die äußere Schicht 3 besonders gut miteinander verbinden und so ein Delaminieren der inneren und der äußeren Schicht 3 verhindert werden. Und zwar, weil die Grenzfläche zwischen der inneren und der äußeren Schicht 3 gegenüber dem Stand der Technik durch die wechselseitige Verzahnung 6 vergrößert ist. Zudem schafft die Geometrie des wechselseitigen Verzahnens eine besonders stabile und beständige mechanische Verbindung zwischen den Schichten 2,3,4, die ein Übertragen von Kräften von der einen in die andere Schicht 2,3,4 besonders sicher und dauerhaft ermöglicht, wodurch - insbesondere bei flexiblen Endoskopschäften 1 - unerwünschtes Delaminieren verhindert wird.

Weiterhin betrifft die Erfindung ein Verfahren zur Herstellung eines solchen erfindungsgemäßen Endoskopschaftes 1.

## Beschreibung

### TECHNISCHES GEBIET

Die Erfindung betrifft einen Endoskopschaft mit einem schichtförmigen Aufbau und ein Verfahren zur Herstellung eines solchen.

### STAND DER TECHNIK

Aus dem deutschen Patent DE 44 38 944 C2 ist ein mindestens abschnittsweise flexibles Endoskop bekannt, das einen schichtförmig aufgebauten Endoskopschaft zeigt. Es weist einen rohrförmigen Schaft aus einer Nickel-Titan-Legierung und ein darin angeordnetes, starres Rohrstück mit einem darin angeordneten Objektiv auf, wobei zwischen dem rohrförmigen Schaft und dem starren Rohrstück ein Lichtleiter angeordnet ist. Dieser Endoskopschaft zeigt einen komplizierten Aufbau mit einer Reihe voneinander getrennten Komponenten, die aufeinander aufwendig abgestimmt werden müssen, um eine verlässliche und dauerhafte Struktur des Endoskopschaftes zu gewährleisten.

Dem Europäischen Patent EP 1 891 881 B1 ist ein Endoskopschaft mit variabler Flexibilität zu entnehmen. Der Schaft zeigt eine flexible, äußere Schicht und eine innere Schicht, wobei die innere Schicht ein Flechtwerk zur Stabilisierung und die flexible, äußere Schicht Dehnungselemente aufweist, die gesteuert durch den Benutzer ihre Längsausdehnung verändern können und dadurch eine Veränderung der Krümmung der flexiblen, äußeren Schicht gegenüber der inneren Schicht und damit des Endoskopschaftes bewirken können. Diese Endoskopschäfte zeigen nach längerer Benutzung und starker Biegebeanspruchung das Risiko einer Delaminierung und damit den Verlust der kontrollierten Einstellung der Krümmung des Endoskopschaftes.

### BESCHREIBUNG DER ERFINDUNG

Der Erfindung liegt die Aufgabe zu Grunde, einen gegenüber dem Stand der Technik verbesserten Schaft für ein Endoskop, anzugeben, der sich durch eine besondere Beständigkeit auszeichnet. Eine weitere Aufgabe besteht darin, ein Verfahren zur Herstellung eines solchen Endoskopschaftes mit einem schichtförmigen Aufbau anzugeben.

Eine Aufgabe wird erfindungsgemäß zum einen durch einen Endoskopschaft mit einem schichtförmigen Aufbau gelöst, welcher die im Anspruch 1 angegebenen Merkmale aufweist. Die andere Aufgabe zur Herstellung der Erfindung wird durch ein Verfahren gelöst, welches die im Anspruch 10 angegebenen Merkmale aufweist.

Vorteilhafte Ausgestaltungen der Erfindung sind Gegenstand der Unteransprüche.

Der erfindungsgemäße Endoskopschaft zeigt einen schichtförmigen Aufbau, bei dem in der Art eines gekrümmten oder nicht gekrümmten Hohlzylinders verschiedene Schichten radial über die gesamte Länge oder zumindest über einen wesentlichen Teil der Länge des Schaftes aufeinanderfolgen und sich schichtweise umschließen. Der schichtförmig aufgebaute Schaft zeigt zumindest eine innere Schicht aus Kunststoff und eine die innere Schicht umschließende äußere Schicht aus Kunststoff sowie eine strukturgebende Schicht im Übergangsbereich der inneren Schicht und der äußeren Schicht. Dabei sind erfindungsgemäß die Kunststoffe der inneren Schicht und der äußeren Schicht unterschiedlich gewählt und die innere Schicht und die äußere Schicht im Übergangsbereich ineinander verzahnt ausgebildet. Hierdurch gelingt es, die innere Schicht und die äußere Schicht besonders gut miteinander zu verbinden und dadurch ein Delaminieren der inneren und der äußeren Schicht zu verhindern. Dies wird erfindungsgemäß dadurch erreicht, dass die Grenzfläche zwischen der inneren Schicht und der äußeren Schicht gegenüber dem Stand der Technik vergrößert ausgebildet ist, was durch die wechselseitige Verzahnung erreicht wird, und zum anderen wird durch die Geometrie des wechselseitigen Verzahnens eine besonders stabile und beständige, mechanische Verbindung zwischen den Schichten geschaffen, die ein Übertragen von Kräften von der einen in die andere Schicht besonders sicher und dauerhaft ermöglicht und dadurch das unerwünschte Delaminieren verhindert. Dies erweist sich gerade bei flexiblen Endoskopschäften als sehr wichtig.

In einer besonders bevorzugten Ausbildung des erfindungsgemäßen Endoskopschaftes zeigt der Kunststoff der inneren Schicht eine niedrigere insbesondere eine mindestens 20°C oder vorzugsweise mindestens 30°C niedrigere Schmelztemperatur als der Kunststoff der äußeren Schicht. Dadurch wird es möglich, dass beim Aufbringen der äußeren Schicht beispielsweise durch Coextrusion auf die innere Schicht diese zum Aufschmelzen gebracht wird und diese beiden aufgeschmolzenen Schichten ineinander schmelzen, ohne dass sich die Kunststoffe der beiden Schichten chemisch verändern. Alternativ oder ergänzend kann das Aufschmelzen der beiden Schichten im Übergangsbereich durch Einbringen von zusätzlicher Energie beispielweise durch Bestrahlen mittels Infrarotstrahlung, Ultraschall oder Beaufschlagung mit elektrischer Energie insbesondere durch Wechselfelder erreicht werden. Durch das Ineinander-Verschmelzen der inneren und der äußeren Schicht aus den verschiedenen Kunststoffen entsteht die gewünschte Verzahnung der beiden Schichten auf besonders einfache Weise, was die gewünschte Beständigkeit der beiden Schichten und damit des Endoskopschaftes bewirkt.

Es hat sich als besonders vorteilhaft erwiesen, den Kunststoff der inneren Schicht so zu wählen, dass seine Schmelztemperatur mindestens 20°C oder vorzugsweise mindestens 30°C niedriger liegt als die des Kunststoffs der äußeren Schicht. Durch diesen deutlichen Schmelztemperaturunterschied lässt sich das gewünschte Ineinander-Verschmelzen der inneren und der äußeren Schicht besonders sicher erreichen.

Bei einer besonders bevorzugten Ausbildung des erfindungsgemäßen Endoskopschaftes wird der Kunststoff der äußeren Schicht des erfindungsgemäßen Endoskopschaftes als Polyether-Block-Amid-Block-Copolymer insbesondere als PEBAX und/oder als Glykol-modifiziertes Poly-Cyclohexylendimethylen-Terephthalat (PCTG) gewählt. Diese Kunststoffe erweisen sich als besonders geeignet für den Einsatz als wesentlicher Bestandteil eines flexiblen Endoskopschaftes insbesondere der äußeren Schicht des Endoskopschaftes. Dies ist im besonderen Maße bedingt durch die Beständigkeit dieser Kunststoffe sowohl in mechanischer als auch in chemischer und biochemischer Hinsicht und im Hinblick auf die Langzeitstabilität. Gerade diese spezifischen Kunststoffe insbesondere PEBAX beziehungsweise Glykol-modifiziertes Poly-Cyclohexylendimethylen-Terephthalat (PCTG) ermöglichen im besonderen Maße den medizinischen Einsatz bei einem flexiblen Endoskop in Verbindung mit einer erfindungsgemäß ausgebildeten und verbundenen inneren Schicht.

Für diese innere Schicht des erfindungsgemäßen Endoskopschaftes werden dabei bevorzugt ein oder mehrere Kunststoffe aus Polyurethan (PU), Thermoplastischem Polyurethan (TPU) und/oder aus Styrol-Block-Copolymer insbesondere Styrol-Ethylen-Butylen-Styrol (SEBS) gewählt. Diese Kunststoffe erweisen sich einerseits durch ihre jeweiligen Schmelztemperaturen als geeignete Partner zu den vorgenannten besonders bevorzugten Kunststoffen für die äußere Schicht und zum anderen durch ihre Beständigkeit und einfache Handhabung als besonders geeignet für die innere Schicht, in der regelmäßig Elemente zur Erhöhung der mechanischen Stabilität und/oder zur Deformation und/oder zur Veränderung der Länge und/oder Elemente zur Überwachung der Eigenschaften (Temperatur, Spannung, Position und Ähnliches) eingebracht sind. Besonders bewährt haben sich dabei die Kunststoffkombinationen für die äußere und die innere Schicht aus PEBAX und Polyurethan (PU) beziehungsweise Glykol-modifiziertem Poly-Cyclohexylendimethylen-Terephthalat (PCTG) und Styrol-Ethylen-Butylen-Styrol (SEBS).

Nach einer bevorzugten Weiterbildung des erfindungsgemäßen Endoskopschaftes ist die strukturgebende Schicht mit einer Vielzahl an Löchern, die durch Stege voneinander getrennt sind, versehen. Erfindungsgemäß ragt durch diese Löcher wenigstens ein Kunststoff der inneren Schicht und/oder der äußeren Schicht hindurch und bildet zumindest einen Teil der ineinander verzahnten inneren und der äußeren Schicht. Diese strukturgebende Schicht ermöglicht es, durch ihre Löcher und Stege das Maß der Verzahnung der inneren und der äußeren Schicht zu steuern.

Dies erfolgt im besonderen Maße auch durch die Positionierung der strukturgebenden Schicht im Übergangsbereich zwischen der inneren und der äußeren Schicht. Insbesondere kann die strukturgebende Schicht eine Grenze des Übergangsbereiches bilden, wenn sich ausschließlich ein Kunststoff von der einen Seite der betreffenden Schicht durch die Löcher in Richtung der anderen Schicht und in diese hinein verzahnend erstreckt. Vorzugsweise erfolgt dies in der inneren Schicht, so dass diese mit ihren strukturgebenden Eigenschaften im besonderen Maß den Hohlraum in dem Endoskopschaft schützt, während gerade bei flexiblen Endoskopen Elemente zur Veränderung der Form insbesondere der Krümmung oder zur Sensierung der Eigenschaften des Endoskops oder der Umgebung eingebracht werden können und dort ihre Funktion aufgrund der Nähe zum Körper besonders vorteilhaft erfüllen können.

Als besonders vorteilhaft hat sich dabei erwiesen, die strukturgebende Schicht im Wesentlichen aus Metall und/oder Kunststoff insbesondere aus Metalldrähten und/oder Kunststofffasern oder aus einem löchrigen Gewebe aus Kunststofffasern und/oder Metalldrähten zu bilden. Dabei hat es sich besonders bewährt, strukturierte Folien aus Kunststoff (insbesondere aus Polyethylen (PE), Polypropylen (PP), Polytetrafluorethylen (PTFE, Teflon), Polystyrol (PS), Polycarbonat (PC) oder Polyvinylidenfluorid (PVDF)) oder aus Metall (insbesondere aus Stahl, medizinischen Stählen, Aluminium, Titan) oder aus hybriden Materialien mit Kunststoff oder Metall zu wählen, die durch geeignete Wahl der Materialstärke, der Materialien oder der Häufigkeit, Lage und Ausbildung der Löcher (insbesondere rund, oval, eckig, in Längsrichtung des Endoskopschaftes präferiert ausgedehnt) besonders die Erfordernisse eines strukturgebenden Elementes für eine strukturgebende Schicht eines erfindungsgemäßen Endoskopschaftes erfüllen können.

Neben diesen Folien haben sich auch Strukturen aus Metalldrähten und/oder Kunststofffasern insbesondere als ein löchriges Gewebe aus Kunststofffasern und/oder Metalldrähten bewährt. Gerade die Gewebe ermöglichen es, sehr vielfältig und präzise die Eigenschaften der strukturgebenden Schicht insbesondere im Hinblick auf die Anordnung und Ausbildung der Löcher für das Durchdringen eines Kunststoffes zum Ineinander-Verzahnen spezifisch zu wählen und dies zu optimieren, ohne die strukturgebende Bedeutung für den endoskopischen Schaft wesentlich zu vernachlässigen. Dabei hat sich eine strukturgebende Schicht aus einem geflochtenen Gewebe mit regelmäßig angeordneten Löchern insbesondere aus Edelstahlfäden oder Kunststofffasern insbesondere aus Polyethylen (PE), Polypropylen (PP), Polyamid (PA, Nylon) Polyvinylchlorid (PVC), Poly-phenylenterephthalamid) (PPTA, Kevlar) besonders bewährt.

Bevorzugt zeigen dabei die Löcher der strukturgebenden Schicht eine Größe von über 0,1 mm, bevorzugt 0,2 mm insbesondere 0,5 mm oder 1 mm und insbesondere Stege der strukturgebenden Schicht eine Breite von über 0,1 mm, bevorzugt 0,2 mm oder auch über 0,5 mm. Durch diese Wahl gelingt es, das Durchdringen und Ineinander-Verzahnen der verschiedenen Kunststoffe besonders gut sicherzustellen und zudem gerade bei der Verwendung von geflochtenem Gewebe als strukturgebende Schicht das Gewicht des Endoskopschaftes gering zu halten, ohne dass die Beständigkeit und die Stabilität des Endoskopschaftes wesentlich beeinträchtigt sind. Dabei hat es sich besonders bewährt, das Verhältnis zwischen der Größe der Stege zu der Größe der Löcher im Bereich oder kleiner als 0,5 zu wählen. Dabei sind die Löcher und auch die Stege, jeweils insbesondere in ihrer Größe, an die Viskosität der beiden Materialien der inneren bzw. äußeren Schicht sowie auch deren Mischung anzupassen.

Darüber hinaus hat es sich als besonders vorteilhaft erwiesen, die innere Schicht und die äußere Schicht im Übergangsbereich mäanderförmig ineinander verzahnt auszubilden. Dies wird insbesondere dadurch erreicht, dass beide Kunststoffe im Übergangsbereich so verflüssigt werden, dass sie sich wechselseitig, mäandrierend ineinander verzahnen und dadurch eine besonders stark vergrößerte wechselseitige Grenzfläche ausbilden, die damit einerseits nach dem Erkalten eine sehr hohe wechselseitige Adhäsion bewirken und zudem durch das wechselseitige, mäanderförmige Verzahnen eine wechselseitige Verschiebung und damit ein Trennen voneinander (Delaminieren) weitgehend ausschließen und damit eine besondere Beständigkeit auch über längere Zeit ermöglichen.

Bei einer bevorzugten Weiterbildung des erfindungsgemäßen Endoskopschaftes ist die äußere Schicht von wenigstens einer Außenschicht umschlossen und/oder die innere Schicht so ausgebildet, dass sie wenigstens eine Innenschicht umschließt. Diese zusätzlichen Schichten sind zum Verbessern der oberflächlichen Eigenschaften des Endoskopschaftes, sei es auf der Außenseite und damit gegenüber einem Körper, in den der Endoskopschaft eingebracht ist, oder gegenüber dem im Innern des Endoskopschaftes angeordneten Hohlraum, der zum Einbringen von endoskopischen Werkzeugen in den Körper verwendet wird, vorgesehen. Dabei werden die Außenschichten insbesondere zur Optimierung der Gleiteigenschaften mit einem geringen Reibungsindex und zur Sicherung der sterilen und/oder biokompatiblen Eigenschaften des Endoskops gewählt und aufgebracht, während die Innenschichten besonders mechanisch robust gewählt sind. Durch diese dünnen zusätzlichen Außen- beziehungsweise Innenschichten lassen sich die Eigenschaften des erfindungsgemäßen Endoskopschaftes gerade im Hinblick auf die Beständigkeit und die Anwendbarkeit weiter verbessern.

Neben dem erfindungsgemäßen Endoskopschaft betrifft die Erfindung auch ein Verfahren zur Herstellung eines Endoskopschaftes mit einem schichtförmigen Aufbau. Hierzu wird in einem Schritt eine innere Schicht aus einem ersten Kunststoff gebildet, die typisch die Gestalt eines Hohlzylinders zeigt. Der Hohlzylinder kann einen kreisförmigen Querschnitt, aber auch einen anderen abgeschlossenen, insbesondere ovalen Querschnitt aufweisen. Diese innere Schicht wird erfindungsgemäß mit einer strukturgebenden Schicht versehen, indem auf der Außenfläche der inneren Schicht eine strukturgebende Schicht aufgebracht oder im Bereich der Außenfläche der inneren Schicht eine strukturgebende Schicht eingebracht wird und anschließend auf der Außenseite der inneren Schicht mit der strukturgebenden Schicht eine äußere Schicht aus einem zweiten Kunststoff aufgebracht wird, der eine höhere Schmelztemperatur als der erste Kunststoff zeigt. Dabei erfolgt dies erfindungsgemäß so, dass die innere Schicht und die äußere Schicht ineinander fließen und dadurch ein Ineinander-Verzahnen der beiden Schichten entsteht, wodurch nach dem Aushärten eine sehr beständige und feste Verbindung der inneren Schicht mit der äußeren Schicht geschaffen ist. Dies wird erfindungsgemäß gerade durch die Verwendung der unterschiedlichen Kunststoffe mit den unterschiedlichen Schmelztemperaturen ermöglicht, da beim Aufbringen der äußeren Schicht im geschmolzenen Zustand die innere Schicht erfindungsgemäß mit aufschmilzt und so das erfindungsgemäße Ineinander-Verzahnen der beiden Schichten ermöglicht wird. Ein Delaminieren der beiden Schichten kann dadurch weitgehend ausgeschlossen werden, denn durch den erfindungsgemäßen Herstellungsprozess gelingt es, die Adhäsionskräfte zwischen der inneren Schicht und der äußeren Schicht durch das Vergrößern der Grenzfläche zwischen den beiden Schichten deutlich zu erhöhen und durch die geänderte Geometrie der Grenzfläche durch das Ineinander-Verzahnen der beiden Schichten ein Trennen der Schichten erheblich zu erschweren. Mithin gelingt es, die Beständigkeit des erfindungsgemäß hergestellten Endoskopschaftes zu erhöhen.

Bei einer vorteilhaften Weiterbildung des erfindungsgemäßen Verfahrens wird während oder nach dem Aufbringen der äußeren Schicht die innere und die äußere Schicht insbesondere mit Infrarotstrahlung und/oder Ultraschall aufgeheizt. Durch das zusätzliche Einbringen von thermischer Energie gelingt es, den Prozess des Ineinander-Verzahnens der inneren und der äußeren Schicht spezifisch zu führen und effizient zu steuern, so dass die Kunststoffe der inneren Schicht und der äußeren Schicht keinen Schaden erleiden, sich gut ineinander verzahnen und die Grenzfläche wirksam vergrößert wird und dadurch die Adhäsionskräfte zwischen den beiden Schichten deutlich vergrößert werden können. Dabei erfolgt das Aufheizen insbesondere lokal begrenzt im Übergangsbereich zwischen den beiden Schichten, der inneren und der äußeren Schicht, so dass diese beiden Schichten verlässlich und räumlich begrenzt ineinanderfließen und sich dadurch ineinander verzahnen können.

Bei einer bevorzugten Weiterbildung des erfindungsgemäßen Verfahrens zur Herstellung eines Endoskopschaftes mit einem schichtförmigen Aufbau wird die innere Schicht durch Coextrusion auf eine entnehmbare Seele gebildet. Gerade die Verwendung des Coextrusionsverfahrens zur Bildung der inneren Schicht, ermöglicht es, die innere Schicht sehr präzise auf der Seele in der Art eines Hohlzylinders auszubilden, wodurch die Voraussetzungen für eine gute Verbindung mit der später aufzubringenden äußeren Schicht zur Bildung der Verzahnung im besonderen Maß gegeben sind. Für die innere Schicht des erfindungsgemäßen Endoskopschaftes werden dabei bevorzugt ein oder mehrere Kunststoffe aus Polyurethan (PU), Thermoplastischem Polyurethan (TPU) und/oder aus Styrol-Block-Copolymer insbesondere Styrol-Ethylen-Butylen-Styrol (SEBS) gewählt.

Darüber hinaus hat es sich als ein besonders vorteilhaftes Verfahren zur Herstellung eines Endoskopschaftes mit einem schichtförmigen Aufbau erwiesen, die strukturgebende Schicht durch Flechten zu bilden, indem ein Flechtwerk mit vielen Löchern auf der Außenfläche oder im Bereich der Außenfläche der inneren Schicht eingebracht wird. Das Ausbilden der strukturgebenden Schicht als Flechtwerk zur Integration in den Übergangsbereich zwischen der inneren Schicht und der äußeren Schicht des Endoskopschaftes erweist sich als sehr vorteilhafter Kompromiss zwischen Stabilität, Flexibilität und Gewicht, was gerade für einen Einsatz bei flexiblen Endoskopen besonders relevant ist. Dabei hat sich eine strukturgebende Schicht aus einem geflochtenen Gewebe mit regelmäßig angeordneten Löchern insbesondere aus Edelstahlfäden oder Kunststofffasern insbesondere aus Polyethylen (PE), Polypropylen (PP), Polyamid (PA, Nylon) Polyvinylchlorid (PVC), Polyphenylenterephthalamid) (PPTA, Kevlar) besonders bewährt.

Bei einer besonders bewährten Weiterbildung eines erfindungsgemäßen Verfahrens zur Herstellung eines Endoskopschaftes mit einem schichtförmigen Aufbau wird die äußere Schicht durch Coextrusion bei einer Verarbeitungstemperatur höher als die Schmelztemperatur der inneren Schicht auf die innere Schicht mit der strukturgebenden Schicht aufgebracht. Dadurch wird eine Verzahnung der aufgeschmolzenen inneren Schicht mit der äußeren Schicht ermöglicht, die erfindungsgemäß eine sehr sichere und dauerhafte Verbindung der inneren und äußeren Schicht des Endoskopschaftes ermöglicht und dadurch für eine ausgesprochene Beständigkeit sorgt. Gerade die Verwendung des Coextrusionsverfahrens zur Bildung der äußeren Schicht auf der inneren Schicht mit der strukturgebenden Schicht, ermöglicht es, die innere Schicht mit der äußeren Schicht so zu verbinden, dass sich beide im flüssigen Zustand verlässlich ineinander verzahnen. Bei dieser besonders bevorzugten Ausbildung der Erfindung wird der Kunststoff der äußeren Schicht als Polyether-Block-Amid-Block-Copolymer insbesondere als PEBAX und/oder als Glykol-modifiziertes Poly-Cyclohexylendimethylen-Terephthalat (PCTG) gewählt.

Besonders bewährt haben sich dabei die Kunststoffkombinationen für die äußere und die innere Schicht aus PEBAX und Polyurethan (PU) beziehungsweise Poly-Cyclohexylendimethylen-Terephthalat (PCTG) und Styrol-Ethylen-Butylen-Styrol (SEBS). Diese Kunststoffkombinationen ermöglichen einerseits ein gutes Ineinanderfließen und damit Ineinander-Verzahnen der beiden Schichten bei dem Aufbringen der äußeren Schicht, was zu einem Aufschmelzen der inneren Schicht führt. Sie ermöglichen zudem eine gute adhäsive Verbindung zwischen den beiden verzahnten Schichten, was sich in einem besonders beständigen durch das erfindungsgemäße Verfahren hergestellten Endoskopschaft zeigt.

In einem besonders bevorzugten erfindungsgemäßen Verfahren zur Herstellung eines Endoskopschaftes mit einem schichtförmigen Aufbau wird wenigstens eine Außenschicht zur Verbesserung der Gleiteigenschaften aufgebracht. Dadurch lassen sich eine bessere Handhabung und eine geringere Belastung des Schaftes erreichen, was zu einer besseren Beständigkeit des hergestellten Endoskopschaftes führt.

Die Erfindung wird nachfolgend anhand bevorzugter Ausführungsbeispiele unter Bezugnahme auf die Abbildungen beispielhaft erläutert. Die Erfindung ist nicht auf diese bevorzugten Ausführungsbeispiele beschränkt.

Es zeigen:
- Fig. 1: eine schematische Darstellung eines beispielhaften erfindungsgemäßen Endoskopschaftes in einem Querschnitt
- Fig. 2: eine schematische Darstellung eines beispielhaften erfindungsgemäßen Endoskopschaftes in einem Längsschnitt und
- Fig. 3: eine schematische Darstellung eines Ausschnitts eines beispielhaften Endoskopschaftes.

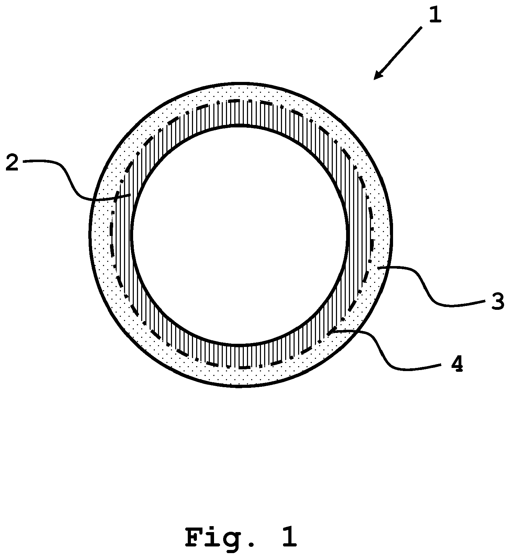

Fig. 1 zeigt in einer schematischen Darstellung einen Querschnitt eines beispielhaften, erfindungsgemäßen, flexiblen Endoskopschaftes 1. Der erfindungsgemäße Endoskopschaft 1 ist in der Art eines gekrümmten oder nicht gekrümmten Hohlzylinders ausgebildet, bei dem verschiedene Schichten radial über die gesamte Länge des Schaftes aufeinanderfolgen und sich schichtweise umschließen. Der schichtförmig aufgebaute Schaft zeigt eine innere Schicht 2 aus einem Kunststoff und eine die innere Schicht 2 umschließende äußere Schicht 3 aus einem anderen Kunststoff sowie eine strukturgebende Schicht 4 im Übergangsbereich 5 der inneren Schicht 2 und der äußeren Schicht 3. Dabei sind erfindungsgemäß die Kunststoffe der inneren Schicht 2 und der äußeren Schicht 3 unterschiedlich gewählt und die innere Schicht 2 und die äußere Schicht 3 im Übergangsbereich 5 ineinander verzahnt ausgebildet. Diese Verzahnung ist in Fig. 1 nicht dargestellt. Durch die Verzahnung gelingt es, die innere Schicht 2 und die äußere Schicht 3 besonders gut miteinander zu verbinden und dadurch ein Delaminieren der inneren Schicht 2 und der äußeren Schicht 3 zu verhindern. Dies wird erfindungsgemäß dadurch erreicht, dass die Grenzfläche zwischen der inneren Schicht 2 und der äußeren Schicht 3 gegenüber dem Stand der Technik vergrößert ausgebildet ist, was durch die wechselseitige Verzahnung 6 erreicht wird, und zum anderen wird durch die Geometrie des wechselseitigen Verzahnens eine besonders stabile und beständige, mechanische Verbindung zwischen den Schichten 2,3 geschaffen, die ein Übertragen von Kräften von der einen Schicht 2,3 in die andere Schicht 3,2 besonders sicher und dauerhaft ermöglicht und dadurch das unerwünschte Delaminieren verhindert. Dies erweist sich gerade bei flexiblen Endoskopschäften als sehr wichtig.

Im Übergangsbereich 5 zwischen der inneren Schicht 2 und der äußeren Schicht 3 ist die strukturgebende Schicht 4 angeordnet und umschließt in der Art eines geschlossenen Ringes die innere Schicht 2, die wiederum zusammen mit der strukturgebenden Schicht 4 in der Art eines geschlossenen Ringes von der äußeren Schicht 3 umschlossen sind.

Die strukturgebende Schicht 4 zeigt eine Vielzahl von Löchern, die durch Stege voneinander getrennt sind. Durch die Löcher kann ein Kunststoff der inneren Schicht 2 beziehungsweise der äußeren Schicht 3 hindurchtreten und in die andere Schicht, in die äußere Schicht 3 beziehungsweise in die innere Schicht 2 hineinfließen und dadurch ein Ineinander-Verzahnen der beiden Schichten 2,3 bewirken. Durch dieses Verzahnen ist eine sehr robuste und beständige, feste Verbindung der inneren Schicht 2 und der äußeren Schicht 3 sowie der strukturgebenden Schicht 4 geschaffen.

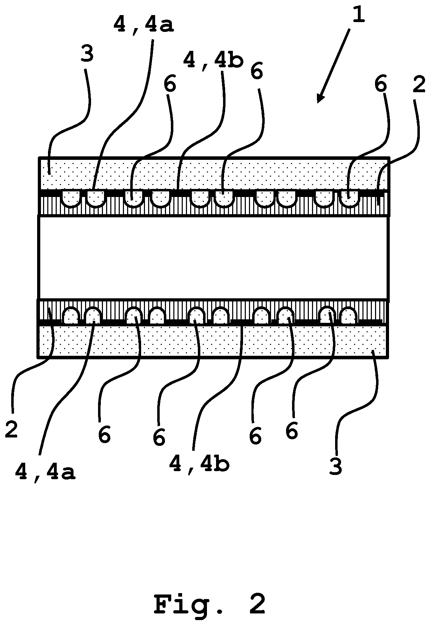

Fig. 2 zeigt in einer schematischen Darstellung einen Längsschnitt eines beispielhaften, erfindungsgemäßen, flexiblen Endoskopschaftes 1 aus Fig. 1. Die strukturgebende Schicht 4 zeigt mehrere Löcher 4a, die regelmäßig, aber mit differenziertem Abstand zueinander über die Länge und den Umfang der strukturgebenden Schicht 4 angeordnet sind. Die Löcher 4a sind voneinander durch Stege 4b getrennt, die unterschiedliche Breite aufweisen. Die strukturgebende Schicht 4 bildet eine Grenze zwischen der inneren Schicht 2 und der äußeren Schicht 3, wobei durch die Löcher 4a Kunststoff der äußeren Schicht 3 in die innere Schicht 2 hindurchgetreten ist und dadurch Ausstülpungen der äußeren Schicht 3 in die innere Schicht 2 gebildet sind, die die Verzahnungen 6 in der inneren Schicht 2 bilden. Durch diese Verzahnungen 6 ist die Oberfläche zwischen der inneren Schicht 2 und der äußeren Schicht 3 vergrößert und dadurch die Kraft zwischen den beiden Schichten durch die zwischen ihnen wirkenden Adhäsionskräfte erhöht. Zu diesem Effekt tritt die Wirkung durch die veränderte, verzahnte Geometrie der Schichten hinzu, so dass eine besonders stabile und beständige Verbindung der inneren Schicht 2 und der äußeren Schicht 3 gegeben ist.

Die strukturgebende Schicht 4 ist dabei als ein löchriges geflochtenes Gewebe aus Metalldrähten ausgebildet. Gerade die Gewebe ermöglichen es, sehr vielfältig und präzise die Eigenschaften der strukturgebenden Schicht 4 insbesondere im Hinblick auf die Anordnung und Ausbildung der Löcher 4a für das Durchdringen eines Kunststoffes zum Ineinander-Verzahnen spezifisch zu wählen und dies zu optimieren, ohne die strukturgebende Bedeutung für den endoskopischen Schaft wesentlich zu vernachlässigen. Dabei hat sich eine strukturgebende Schicht 4 aus einem geflochtenen Gewebe mit regelmäßig angeordneten Löchern 4a insbesondere aus Edelstahlfäden besonders bewährt.

Dabei zeigen die kreisrunden Löcher 4a der strukturgebenden Schicht 4 eine Größe von über 0,1 mm, bevorzugt 0,2 mm insbesondere 0,5 mm oder 1 mm und die Stege 4b der strukturgebenden Schicht 4 eine Breite von etwa 0,1 mm, bevorzugt 0,2 mm oder auch über 0,5 mm. Durch diese Wahl gelingt es, das Durchdringen und Ineinander-Verzahnen der verschiedenen Kunststoffe besonders gut sicherzustellen und zudem gerade bei der Verwendung von geflochtenem Gewebe als strukturgebende Schicht 4 das Gewicht des Endoskopschaftes 1 gering zu halten, ohne dass die Beständigkeit und die Stabilität des Endoskopschaftes 1 wesentlich beeinträchtigt sind. Dabei hat es sich besonders bewährt, das Verhältnis zwischen der Größe der Stege 4b zu der Größe der Löcher 4a im Bereich von 0,5 zu wählen.

Bei dieser Ausbildung des erfindungsgemäßen Endoskopschaftes 1 wird als Kunststoff für die äußere Schicht 3 des erfindungsgemäßen Endoskopschaftes 1 PEBAX gewählt. Dieser Kunststoff erweist sich als besonders geeignet für den Einsatz als wesentlicher Bestandteil eines flexiblen Endoskopschaftes 1 insbesondere der äußeren Schicht 3 des Endoskopschaftes 1. Dies ist im besonderen Maße bedingt durch die Beständigkeit dieses Kunststoffes sowohl in mechanischer als auch in chemischer und biochemischer Hinsicht und im Hinblick auf die Langzeitstabilität. Gerade dieser spezifische Kunststoff PEBAX ermöglicht im besonderen Maße den medizinischen Einsatz bei einem flexiblen Endoskop in Verbindung mit einer erfindungsgemäß ausgebildeten und verbundenen inneren Schicht 2, da dieser eine Zulassung für Medizinprodukte zeigt und sich zusätzlich durch einen geringen Gleitreibungsindex auszeichnet.

Für diese innere Schicht 2 des in Fig. 2 dargestellten Endoskopschaftes 1 wird als Kunststoff Polyurethan (PU) gewählt. Dieser erweist sich durch seine Schmelztemperatur als geeigneter Partner zu dem vorgenannten Kunststoff PEBAX für die äußere Schicht 3. Er zeigt dabei eine um mehr als 20°C oder vorzugsweise 30°C niedrigere Schmelztemperatur als der für die äußere Schicht 3 gewählte Kunststoff PEBAX. Er erweist sich zum anderen durch seine Beständigkeit und einfache Handhabung als besonders geeignet für die innere Schicht 2, in der regelmäßig Elemente zur Erhöhung der mechanischen Stabilität und/oder zur Deformation und/oder zur Veränderung der Länge und/oder Elemente zur Überwachung der Eigenschaften (Temperatur, Spannung, Position und Ähnliches) eingebracht sind.

Gerade diese Kombination an Kunststoffen für die innere Schicht 2 und die äußere Schicht 3 erweist sich als besonders vorteilhaft, da es möglich ist, die erfindungsgemäße Verzahnung 6 im Rahmen des Aufbringens der äußeren Schicht 3 auf den Verbund aus innerer Schicht 2 mit strukturgebender Schicht 4 durch Coextrusion, was bei einer Temperatur im Bereich der Schmelztemperatur von PEBAX erfolgt, zu schaffen, denn bei dieser Arbeitstemperatur schmilzt die innere Schicht aus PU teilweise mit auf, so dass die verschiedenen Kunststoffe ineinanderfließen können, wodurch ein Ineinander-Verzahnen der beiden Schichten 2,3 entsteht.

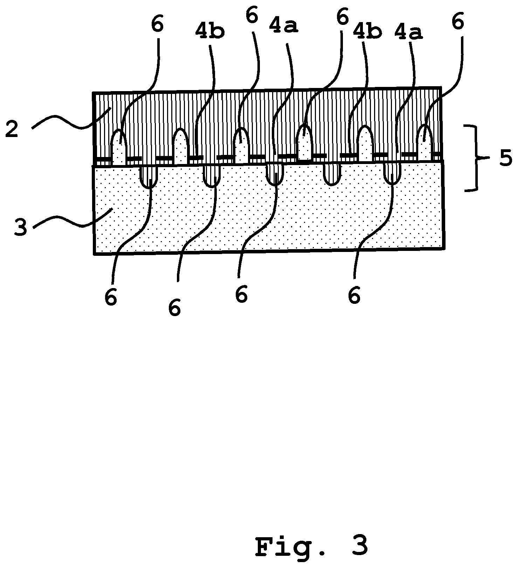

In Fig. 3 ist in einer schematischen Darstellung ein Ausschnitt eines anderen beispielhaften Endoskopschaftes 1 dargestellt. Sie zeigt die innere Schicht 2, an die sich die äußere Schicht 3 anschließt.

In der inneren Schicht 2, beabstandet von der äußeren Schicht 3, erstreckt sich über die Länge und den Umfang der inneren Schicht 2 die strukturgebende Schicht 4, die durch eine strukturierte Folie aus dem Kunststoff Polyvinylidenfluorid (PVDF) gebildet ist. Die Materialstärke, die Häufigkeit, die Lage und die Ausbildung der Löcher wird so gewählt, dass eine ausreichende Stabilität, ein möglichst geringes Gewicht und eine gute Verzahnung der Kunststoffe der inneren Schicht 2 und der äußeren Schicht 3 für den erfindungsgemäßen Endoskopschaft 1 erreicht werden.

Bei dieser Ausbildung dieses erfindungsgemäßen Endoskopschaftes 1 wird als Kunststoff für die äußere Schicht 3 des erfindungsgemäßen Endoskopschaftes 1 Glykol-modifiziertes Poly-Cyclohexylendimethylen-Terephthalat (PCTG) gewählt. Für die innere Schicht 2 dieses in Fig. 3 dargestellten Endoskopschaftes 1 wird als Kunststoff Styrol-Ethylen-Butylen-Styrol (SEBS) gewählt. Dieser erweist sich durch seine Schmelztemperatur im Bereich zwischen 150°C und 210°C als geeigneter Partner zu dem vorgenannten Kunststoff Glykol-modifiziertes Poly-Cyclohexylendimethylen-Terephthalat (PCTG) mit einer Schmelz- beziehungsweise Verarbeitungstemperatur von etwa 240°C als besonders geeigneter Kunststoffpartner. Auch erweist sich Styrol-Ethylen-Butylen-Styrol (SEBS) durch seine Beständigkeit und einfache Handhabung als besonders geeignet für die innere Schicht 2. Insbesondere erweist er sich auch als besonders geeignet, die vorgenannte strukturierte Kunststofffolie als strukturierte Schicht 4 durch Umspritzen mittels Coextrusion aufzunehmen.

Beim Aufbringen der äußeren Schicht 3 auf die innere Schicht 2 schmilzt die innere Schicht 2 oberflächlich auf, wodurch Ausstülpungen von der inneren Schicht 2 zur äußeren Schicht 3 wie auch umgekehrt von der äußeren Schicht 3 zur inneren Schicht 2 entstehen, die die Verzahnungen 6 bilden. Dabei erstrecken sich einzelne Verzahnungen 6 auch durch die Löcher 4a der strukturgebenden Schicht 4.

Durch das wechselseitige Verzahnen der einen Schicht 2,3 mit der anderen Schicht 3,2 entsteht eine mäanderförmige Struktur von Verzahnungen 6, die sich durch eine ausgesprochen Beständigkeit und feste Verbindung der beteiligten Schichten 2,3,4 auszeichnet. Das unerwünschte Delaminieren der inneren Schicht 2 und der äußeren Schicht 3 ist hierdurch weitgehend ausgeschlossen. In Fig. 3 ist darüber hinaus der Übergangsbereich 5 zwischen der inneren Schicht 2 und der äußeren Schicht 3 dargestellt. Der Übergangsbereich 5 erstreckt sich von der weitesten Ausdehnung der Ausstülpung der inneren Schicht 2 in die äußere Schicht 3 (Verzahnung 6) bis zur weitesten Ausdehnung der Ausstülpung der äußeren Schicht 3 in die innere Schicht 2 (Verzahnung 6). In diesem Übergangsbereich 5 befindet sich die strukturgebende Schicht 4.

Die Erfindung betrifft einen Endoskopschaft 1 mit schichtförmigem Aufbau. Bei diesem als gekrümmter oder nicht gekrümmter Hohlzylinders gebildeten Schaft 1 folgen verschiedene Schichten, sich umschließend, radial über die gesamte oder einen wesentlichen Teil der Länge des Schaftes aufeinander. Der Schaft zeigt eine innere Schicht 2 aus Kunststoff und eine die innere Schicht 2 umschließende äußere Schicht 3 aus Kunststoff sowie eine strukturgebende Schicht 4 im Übergangsbereich 5 der inneren und der äußeren Schicht 3. Dabei sind die Kunststoffe der inneren und äußeren Schicht 3 unterschiedlich und die innere und die äußere Schicht 3 im Übergangsbereich 5 ineinander verzahnt. So können sich die innere und die äußere Schicht 3 besonders gut miteinander verbinden und so ein Delaminieren der inneren und der äußeren Schicht 3 verhindert werden. Und zwar, weil die Grenzfläche zwischen der inneren und der äußeren Schicht 3 gegenüber dem Stand der Technik durch die wechselseitige Verzahnung 6 vergrößert ist. Zudem schafft die Geometrie des wechselseitigen Verzahnens eine besonders stabile und beständige mechanische Verbindung zwischen den Schichten 2,3,4, die ein Übertragen von Kräften von der einen in die andere Schicht 2,3,4 besonders sicher und dauerhaft ermöglicht, wodurch - insbesondere bei flexiblen Endoskopschäften 1 - unerwünschtes Delaminieren verhindert wird.

Weiterhin betrifft die Erfindung ein Verfahren zur Herstellung eines solchen erfindungsgemäßen Endoskopschaftes 1.

### Bezugszeichenliste

- 1.: Endoskopschaft
- 2.: Innere Schicht
- 3.: Äußere Schicht
- 4.: Strukturgebende Schicht
- 4a.: Löcher
- 4b.: Stege
- 5.: Übergangsbereich zwischen innerer und äußerer Schicht
- 6.: Verzahnung

## Patentansprüche

1. Endoskopschaft (1) mit einem schichtförmig aufgebauten Schaft, der eine innere Schicht (2) aus Kunststoff,
der eine die innere Schicht (2) umschließende äußere Schicht (3) aus Kunststoff
und der eine strukturgebende Schicht (4) im Übergangsbereich (5) der inneren Schicht (2) und der äußeren Schicht (3) zeigt,
**dadurch gekennzeichnet,**
**dass** die Kunststoffe der inneren Schicht (2) und der äußeren Schicht (3) unterschiedlich gewählt sind
und **dass** die innere Schicht (2) und die äußere Schicht (3) im Übergangsbereich (5) ineinander verzahnt (6) ausgebildet sind.

2. Endoskopschaft nach Anspruch 1, **dadurch gekennzeichnet, dass** der Kunststoff der inneren Schicht (2) eine niedrigere insbesondere eine mindestens 20°C oder vorzugsweise mindestens 30°C niedrigere Schmelztemperatur als der Kunststoff der äußeren Schicht (3) zeigt.

3. Endoskopschaft nach Anspruch 2, **dadurch gekennzeichnet, dass** der Kunststoff der äußeren Schicht (3) als Polyether-Block-Amid-Block-Copolymer insbesondere als PEBAX und/oder als Glykol-modifiziertes Poly-Cyclohexylendimethylen-Terephthalat (PCTG) gewählt ist und der Kunststoff der inneren Schicht (2) insbesondere aus Polyurethan (PU), Thermoplastisches Polyurethan (TPU) und/oder aus Styrol-Block-Copolymer insbesondere aus Styrol-Ethylen-Butylen-Styrol (SEBS) gewählt ist.

4. Endoskopschaft nach einem der Ansprüche 1 bis 3, **dadurch gekennzeichnet, dass** die strukturgebende Schicht (4) eine Vielzahl an Löchern (4a) zeigt, die durch Stege (4b) voneinander getrennt sind und durch die wenigstens ein Kunststoff der inneren Schicht (2) und/oder der äußeren Schicht (3) hindurchragt und insbesondere einen Teil der ineinander verzahnt (6) ausgebildeten inneren Schicht (2) und der äußeren Schicht (3) bildet.

5. Endoskopschaft nach einem der Ansprüche 1 bis 3, **dadurch gekennzeichnet, dass** die strukturgebende Schicht (4) eine Grenze des Übergangsbereichs (5) zu einer der beiden Schichten (2,3) bildet.

6. Endoskopschaft nach Anspruch 4 oder 5, **dadurch gekennzeichnet, dass** die strukturgebende Schicht (4) im Wesentlichen aus Metall und/oder Kunststoff insbesondere aus Metalldrähten und/oder Kunststofffasern oder aus einem löchrigen Gewebe aus Kunststofffasern und/oder Metalldrähten gebildet ist.

7. Endoskopschaft nach einem der Ansprüche 4 bis 6, **dadurch gekennzeichnet, dass** Löcher (4a) der strukturgebenden Schicht (4) eine Größe von über 0,1 mm, bevorzugt 0,2 mm insbesondere 0,5 mm oder 1 mm zeigen und insbesondere Stege (4b) der strukturgebenden Schicht (4) eine Breite von über 0,1 mm, bevorzugt 0,2 mm, insbesondere über 0,5 mm zeigen.

8. Endoskopschaft nach einem der Ansprüche 1 bis 7, **dadurch gekennzeichnet, dass** die innere Schicht (2) und die äußere Schicht (3) im Übergangsbereich (5) mäanderförmig ineinander verzahnt ausgebildet sind.

9. Endoskopschaft einem der Ansprüche 1 bis 7, **dadurch gekennzeichnet, dass** die äußere Schicht (3) von wenigstens einer Außenschicht umschlossen ist und/oder die innere Schicht (2) wenigstens eine Innenschicht umschließt.

10. Verfahren zur Herstellung eines Endoskopschaftes mit einem schichtförmigen Aufbau,
**dadurch gekennzeichnet,**
**dass** eine innere Schicht (2) aus einem ersten Kunststoff gebildet wird und diese mit einer strukturgebenden Schicht (4) versehen wird, indem auf der Außenfläche oder im Bereich der Außenfläche der inneren Schicht (2) eine strukturgebende Schicht (4) eingebracht wird,
**dass** anschließend auf der Außenseite der inneren Schicht (2) mit der strukturgebenden Schicht (4) eine äußere Schicht (3) aus einem zweiten Kunststoff aufgebracht wird, der eine höhere Schmelztemperatur als der erste Kunststoff zeigt, wobei dies so erfolgt, dass die innere Schicht (2) und die äußere Schicht (3) ineinander verzahnt aushärten.

11. Verfahren zur Herstellung eines Endoskopschaftes mit einem schichtförmigen Aufbau nach Anspruch 10, **dadurch gekennzeichnet, dass** während oder nach dem Aufbringen der äußeren Schicht (3) ein Aufheizen der inneren Schicht (2) und der äußeren Schicht (3) insbesondere mit Infrarotstrahlung und/oder Ultraschall erfolgt.

12. Verfahren zur Herstellung eines Endoskopschaftes mit einem schichtförmigen Aufbau nach einem der Ansprüche 10 bis 11, **dadurch gekennzeichnet, dass** wenigstens eine Außenschicht zur Verbesserung der Gleiteigenschaften aufgebracht wird.

13. Verfahren zur Herstellung eines Endoskopschaftes mit einem schichtförmigen Aufbau nach einem der Ansprüche 10 bis 12, **dadurch gekennzeichnet, dass** die innere Schicht (2) durch Coextrusion auf eine entnehmbare Seele gebildet wird.

14. Verfahren zur Herstellung eines Endoskopschaftes mit einem schichtförmigen Aufbau nach einem der Ansprüche 10 bis 13, **dadurch gekennzeichnet, dass** die strukturgebende Schicht (4) durch Flechten gebildet wird, indem ein Flechtwerk mit vielen Löchern (4a) auf der Außenfläche oder im Bereich der Außenfläche der inneren Schicht (2) eingebracht wird.

15. Verfahren zur Herstellung eines Endoskopschaftes mit einem schichtförmigen Aufbau nach einem der Ansprüche 10 bis 14, **dadurch gekennzeichnet, dass** die äußere Schicht (3) durch Coextrusion bei einer Verarbeitungstemperatur höher als die Schmelztemperatur des Kunststoffes der inneren Schicht (2) auf die innere Schicht (2) mit der strukturgebenden Schicht (4) aufgebracht wird, wodurch eine Verzahnung (6) der aufgeschmolzenen inneren Schicht (2) mit der äußeren Schicht (3) erfolgt.
